(19) Europäisches Patentamt
European Patent Office
Office européen des brevets

(11) EP 0 703 229 A2

(12) # EUROPEAN PATENT APPLICATION

(43) Date of publication:
27.03.1996 Bulletin 1996/13

(51) Int. Cl.[6]: C07D 311/58

(21) Application number: 95118114.8

(22) Date of filing: 08.10.1992

(84) Designated Contracting States:
AT BE CH DE DK ES FR GB GR IE IT LI LU MC NL PT SE

(30) Priority: 08.10.1991 SE 9102905
29.06.1992 SE 9202000

(62) Application number of the earlier application in accordance with Art. 76 EPC: 92921525.9

(71) Applicant: Astra Aktiebolag
S-151 85 Södertälje (SE)

(72) Inventor: Johansson, Lars George
S-15148 Södertälje (SE)

Remarks:
This application was filed on 17 - 11 - 1995 as a divisional application to the application mentioned under INID code 62.

(54) **Process for the preparation of (R)-3-amino-5-methoxychroman**

(57) A process for preparing (R)-3-Amino-5-methoxychroman as well as the product prepared by said process.

EP 0 703 229 A2

## Description

### Field of the Invention

The present invention relates to the new compound 3-(N-isopropyl-N-n-propylamino)-5-(N-isopropyl)carbamoylchroman in the racemic form as well as the (R)-enantiomer thereof in the form of free base or pharmaceutically acceptable salts thereof, process for their preparation, pharmaceutical compositions containing said therapeutically active compound and to the use of said active compound in therapy.

An object of the invention is to provide a compound for therapeutic use, especially a compound having a highly selective affinity for a subgroup of 5-hydroxytryptamine receptors ($5HT_{IA}$) in the central nervous system (CNS) of mammals including man.

It is also an object of the invention to provide a compound with a therapeutic effect after oral administration.

### Prior art

Substituted-3-aminochromans intended for therapeutic use in the central nervous system are disclosed in some patent documents inter alia EP 0 222 996 and WO 91/09853.

### Background of the Invention

Various central nervous system disorders such as depression, anxiety, etc. appear to involve the disturbance of the neurotransmitters noradrenaline (NA) and 5-hydroxytryptamine (5-HT), the latter also known as serotonin. The drugs most frequently used in the treatment of depression are believed to act by improving the neurotransmission of either or both of these physiological agonists. It appears that the enhancement of 5-HT neurotransmission primarily affects the depressed mood and anxiety, whereas the enhancement of noradrenaline neurotransmission affects the retardation symptoms occuring in depressed patients. The invention concerns compounds which have an effect on 5-HT neurotransmission.

Serotonin, or 5-HT, activity is thought to be involved in many different types of psychiatric disorders. For instance it is thought that an increase in 5-HT activity is associated with anxiety, while a decrease in 5-HT release has been associated with depression. Serotonin has in addition been implicated in such diverse conditions as eating disorders, cardiovascular regulation and sexual behavior.

### The 5-HT Receptors

The various effects of serotonin may be related to the fact that serotonergic neurons stimulate the secretion of severeal other hormones, e.g. cortisol, prolactin, β-endorphin, vasopressin and others. The secretion of each of these other hormones appears to be regulated on a specific basis by several different 5-HT (serotonin) receptor subtypes. With the aid of molecular biology techniques, to date these receptors have been classified as $5\text{-}HT_1$, $5\text{-}HT_2$, $5\text{-}HT_3$, and $5\text{-}HT_4$ with the $5\text{-}HT_1$ receptor further divided into the $5\text{-}HT_{1A}$, $5\text{-}HT_{1B}$, $5\text{-}HT_{1C}$ and $5\text{-}HT_{1D}$ subtypes. Each receptor subtype is involved in a different serotonin function and has different properties.

### 5-HT-Receptor-Active Agents

The mechanism of action for the drugs generally used today in the therapy of mental depression is indirect, i.e. they act by blocking the re-uptake of the neurotransmitters (NA and/or 5-HT) released from nerve terminals in the central nervous system. These drugs increase the concentration of the neurotransmitters in the synaptic cleft and hence restore adequate neurotransmission.

A fundamentally different way of improving the neurotransmission in the central nervous system 5-HT neurons would be to use a direct 5-HT-receptor-active agent. In order to minimize side effects, or to effect a specific type of behavior or serotonin function, a high selectivity for a specific 5-HT receptor subtype would be preferred. Agonists can be used to activate specific receptors.

The object of the present invention is to provide compounds for therapeutic use, especially for use in the treatment of 5-hydroxytryptamine mediated disorders in the central nervous system for instance depression, anxiety, obsessive-compulsive disorder (OCD), anorexia, senile dementia, migraine, stroke, Alzheimer's disease, hypertension, thermoregulatory and sexual disturbances, pain and for use in the treatment of disturbances in the cardiovascular system.

The invention

It has turned out that various 3-(N,N-dialkylamino)-5-(N-alkyl)carbamoylchromans tested, show a great variability in the three different parameters bioavailability, 5-$HT_{IA}$ receptor stimulating effect and in selectivity. It has been difficult to identify compounds possessing all three advantageous properties. There is no guidance in the prior art how to obtain compounds with this combination of properties.

Surprisingly, it has been found that the racemic compound of the invention, 3-(N-isopropyl-N-n-propylamino)-5-(N-isopropyl)carbamoylchroman shows excellent bioavailability, and possess a high affinity to a specific sub-group of 5-hydroxytryptamine receptors in CNS, the 5-$HT_{IA}$.

Furthermore, it has been found that high affinity for the 5-$HT_{IA}$-receptor in CNS is strictly stereospecific as regards the compound, 3-(N-isopropyl-N-n-propylamino)-5-(N-isopropyl)carbamoylchroman. The (R)-enantiomer of 3-(N-isopropyl-N-n-propylamino)-5-(N-isopropyl)carbamoylchroman possesses a high affinity for 5-$HT_{IA}$ receptors in CNS while the (S)-enantiomer of 3-(N-isopropyl-N-n-propylamino)-5-(N-isopropyl)carbamoylchroman lacks activity for 5-$HT_{IA}$ receptors. The (R)-enantiomer of 3-(N-isopropyl-N-n-propylamino)-5-(N-isopropyl)carbamoylchroman shows a good bioavailability, too. Thus the racemate as well as the (R)-enantiomer of the compound of the invention can be used in the treatment of 5-hydroxytryptamine mediated states and disorders in mammals including man.

The compound of the present invention is 3-(N-isopropyl-N-n-propylamino)-5-(N-isopropyl)carbamoylchroman as racemate and (R)-enantiomer having the formula

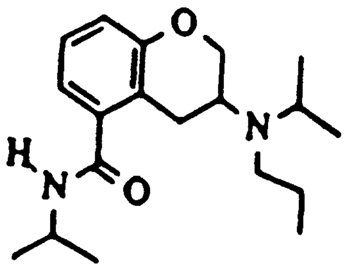

I

in the form of free base or pharmaceutically acceptable salts thereof.

Both organic and inorganic acids can be employed to form non-toxic pharmaceutically acceptable acid addition salts of the compound of this invention. Illustrative acids are sulfuric, nitric, phosphoric, oxalic, hydrochloric, formic, hydrobromic, citric, acetic, lactic, tartaric, dibenzoyltartaric, diacetyltartaric, pamoic, ethanedisulfonic, sulfamic, succinic, propionic, glycollic, malic, gluconic, pyruvic, phenylacetic, 4-aminobenzoic, anthranilic, salicylic, 4-aminosalicylic, 4-hydroxybenzoic, 3,4-dihydroxybenzoic, 3,5-dihydroxybenzoic, 3-hydroxy-2-naphtoic, nicotinic, methanesulfonic, ethanesulfonic, hydroxyethanesulfonic, benzenesulfonic, p-toluenesulfonic, sulfanilic, naphthalenesulfonic, ascorbinic, cyclohexylsulfamic, fumaric, maleic and benzoic acids. These salts are readily prepared by methods known in the art.

Preparation

The (R)-enantiomer may be obtained according to known methods such as from racemic diastereomeric salts by means of fractional crystallisation or covalent diastereomers by means of chromatography. The enantiomeric separation may be performed before alkylation of the amino group (Method A) or after N-alkylation (Method B). The scheme below

illustrates the Methods A and B in more detail:

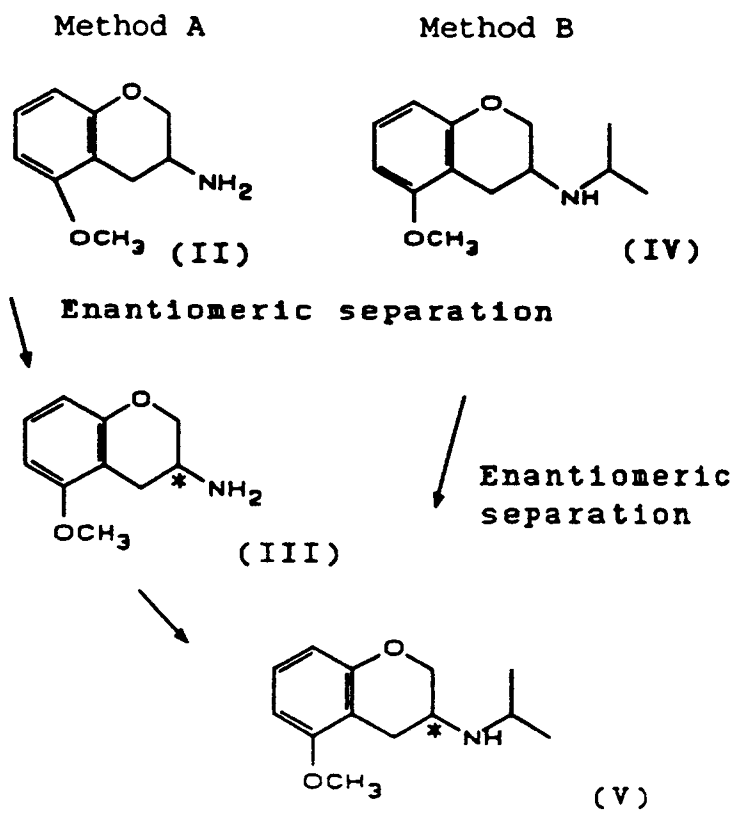


The compound V may be transferred to compound VI by using known method steps such as N-alkylation, demethylation and finally conversion to the leaving group Y.

The preparation of the racemic compound of the invention may start from the compound 5-methoxy-3-chromanone (prepared analogously to the description in EP 222 996) followed by known methods such as reductive amination, N-alkylation, demethylation and finally conversion to the leaving group Y to obtain the racemate of compound VI.

The racemic form as well as the (R)-enantiomer of the compound of the invention, may be prepared according to the following methods:

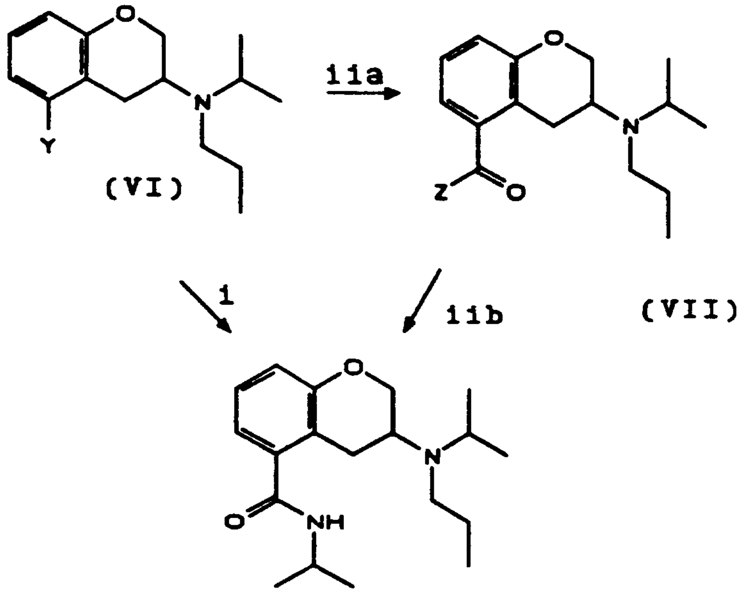


(VI) (VII)

(I)

i, converting the compound of formula VI

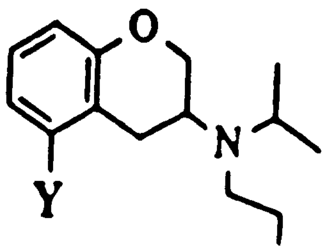

VI

wherein Y is a leaving group such as trifluoromethane sulfonate ($OSO_2CF_3$), halide e.g. Cl, or Br or I by a catalytic cycle using a zerovalent transition metal (M) such as Pd or Ni, which may be generated in situ and undergoes an oxidative addition to the aryl-Y-bond. Treatment with carbon monoxide followed by amination with isopropylamine give the the compound of formula I, whereafter if desired it is converted to a salt.

ii, Alternatively, the compound of formula VI is converted to the compound of formula VII

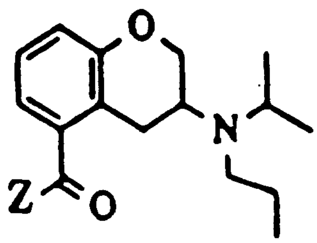

VII

wherein Z is Cl, Br, OH or $OR_p$, where Rp is $C_1$-$C_6$ alkyl, by catalytic cycle using a zerovalent transition metal, with ability to undergo oxidative addition to aryl-Y-bonds e.g. the aryl-$SO_3CF_3$ bonds. The aryl-CO-metal-Y complex is formed by treatment with carbon monoxide (CO).

Further reagents are an alcohol such as methanol, ethanol, a tertiary amine base such as a trialkylamine e.g. triethylamine in an inert organic solvent preferentially a polar aprotic solvent such as dimethylformamide (DMF), dimethylsulfoxide (DMSO), acetone, acetonitrile etc. The reaction is normally performed at a temperature between +40 to +120°C and at a pressure between 100 to 500 KPa (iia). This is optionally followed by hydrolysis and treatment with a thionyl halide, e.g. thionylchloride, to obtain the corresponding acid halide derivative.

The compound of formula VII is aminated (iib) with isopropylamine in a nonpolar aprotic solvent e.g. toluene, benzene at reflux temperature or between 0 to 100°C to give the compound of formula I.

## Pharmaceutical preparations

According to the present invention the compound of the invention will normally be administered orally, rectally or by injection, in the form of pharmaceutical preparations comprising the active ingredient either as a free base or a pharmaceutically acceptable non-toxic acid addition salt, e.g. the hydrochloride, hydrobromide, lactate, acetate, phosphate, sulfate, sulfamate, citrate, tartrate, oxalate and the like in a pharmaceutically acceptable dosage form. The dosage form may be a solid, semisolid or liquid preparation. Usually the active substance will constitute between 0.1 and 99% by weight of the preparation, more specifically between 0.5 and 20% by weight for preparations intended for injection and between 0.2 and 50% by weight for preparations suitable for oral administration.

To produce pharmaceutical preparations containing the compound of the invention in the form of dosage units for oral application, the selected compound may be mixed with a solid excipient, e.g. lactose, saccharose, sorbitol, mannitol, starches such as potato starch, corn starch or amylopectin, cellulose derivatives, a binder such as gelatine or polyvinylpyrrolidone, and a lubricant such as magnesium stearate, calcium stearate, polyethylene glycol, waxes, paraffin, and the like, and then compressed into tablets. If coated tablets are required, the cores, prepared as described above, may be coated with a concentrated sugar solution which may contain e.g. gum arabic, gelatine, talcum, titanium dioxide, and the like. Alternatively, the tablet can be coated with a polymer known to the man skilled in the art, dissolved in a readily volatile organic solvent or mixture of organic solvents. Dyestuffs may be added to these coatings in order to readily distinguish between tablets containing different active substances or different amounts of the active compound.

For the preparation of soft gelatine capsules, the active substance may be admixed with e.g. a vegetable oil or polyethylene glycol. Hard gelatine capsules may contain granules of the active substance using either the above mentioned excipients for tablets e.g. lactose, saccharose, sorbitol, mannitol, starches (e.g. potato starch, corn starch or amylopectin), cellulose derivatives or gelatine. Also liquids or semisolids of the drug can be filled into hard gelatine capsules.

Dosage units for rectal application can be solutions or suspensions or can be prepared in the form of suppositories comprising the active substance in a mixture with a neutral fatty base, or gelatine rectal capsules comprising the active substance in admixture with vegetable oil or paraffin oil. Liquid preparations for oral application may be in the form of syrups or suspensions, for example solutions containing from about 0.2% to about 20% by weight of the active substance herein described, the balance being sugar and mixture of ethanol, water, glycerol and propylene glycol. Optionally such liquid preparations may contain colouring agents, flavouring agents, saccharine and carboxymethyl-cellulose as a thickening agent or other excipients known to the man in the art.

Solutions for parenteral applications by injection can be prepared in an aqueous solution of a water-soluble pharmaceutically acceptable salt of the active substance, preferably in a concentration of from about 0.5% to about 10% by weight. These solutions may also contain stabilizing agents and/or buffering agents and may conveniently be provided in various dosage unit ampoules. Suitable daily doses of the compound of the invention in therapeutical treatment of humans are about 0.01-100 mg/kg bodyweight at peroral administration and 0.001-100 mg/kg bodyweight at parenteral administration.

The invention is illustrated by the following working examples.

Example 1

**3-(N-Isopropyl-N-n-propylamino)-5-(N-isopropyl)carbamoyl chroman**

**a) 3-Isopropylamino-5-methoxychroman hydrochloride**

5-Methoxy-3-chromanone (16 g, 0,089 mol) and isopropylamine (6,4 g, 0,112 mol) were reacted via reductive amination by known mehods (Clinton F. Lane Synthesis 1975 vol. 46 p. 135) to give the title compound. Mp. 255°C.

**b) 3-(N-Isopropyl-N-n-propylamino)-5-methoxychroman**

A mixture of the product obtained in a) above (14 g, 0,06 mol), 1-iodopropane (15 g, 0,08 mol), $K_2CO_3$ and acetonitrile (250 ml) was stirred under reflux for 4 days. After chromatography the desired product was isolated as a colourless oil. GC-MS(CI-mode) M+1 = 264 (100%).

**c) 3-(N-Isopropyl-N-n-propylamino)-5-hydroxychroman**

The product from b) above (10 g, 0,038 mol) was demethylated using $BBr_3$ in dichloromethane.
GC-MS(CI-mode) M+1 = 250 (100%).

**d) 3-(N-Isopropyl-N-n-propylamino)-5-trifluoromethanesulfonoxychroman**

The product from c) above (10 g, 0,04 mol) was dissolved in $CH_2Cl_2$ and cooled to -30°C. Pyridine (6 g, 0,076 mol) was added followed by trifluoromethanesulfonic anhydride (14 g, 0,05 mol). The solution was stirred at -20°C for 3 hours and then allowed to reach ambient temperature. The solution was washed with aqueous $NaHCO_3$, dried with $Na_2SO_4$ and evaporated to dryness. The oil was finally purified by flash chromatography (silica gel) by elution with ethyl acetate/hexane 1:9.
GC-MS(CI-mode) M+1 = 382 (100%)

**e) 3-(N-Isopropyl-N-n-propylamino)-5-(N-isopropyl)carbamoylchroman**

A mixture of the product from d) above (3 g; 0.008 mL), 1,3-bis(diphenylphosphine)propane (150 mg), palladium (II) acetate (75 mg), and isopropylamine (5 ml) in 30 ml DMF was placed in a Parr glass vessel. CO at 2 bar was added and the mixture was shaken at 60°C for 4 hours. After work up and chromatographic purification the desired compound was obtained as white crystals with mp 124°C (base).

Example 2

**(R)-3-(N-Isopropyl-N-n-propylamino)-5-(N-isopropyl)carbamoylchroman (I) (Method A)**

**a) 3-Amino-5-methoxychroman hydrochloride (II)**

3-Amino-5-methoxychroman was prepared according to known methods (Acta Pharm. Suec. 24, 169-182, 1987). Mp: 237-238°C.

**b) (R)-3-Amino-5-methoxychroman (III)**

The racemic 3-amino-5-methoxychroman as base (1.0 g; 5.5 mmol) and L(+)tartaric acid (1.0 g; 6.6 mmol) were dissolved in water (20 ml). The solution was heated to 70°C and the clear solution was allowed to crystallize at room temperature overnight. The precipitate was filtered off giving 0.7 g of the tartrate salt with 99% optical purity of the (R)-isomer. Alkalization and extraction with $CH_2Cl_2$ drying ($Na_2SO_4$) and evaporation gave 0.35 g (35%) of the title compound as the free base.
$[\alpha]_D^{25}$ =-20.8° (C=0.83, $CH_2Cl_2$).

**c) (R)-3-(N-Isopropylamino)-5-methoxychroman (V)**

(R)-3-Amino-5-methoxychroman (1.0 g; 6mmol) and acetone (2.5 g; 40 mmol) were mixed with methanol (820 ml), acetic acid (0.4 g; 6.6 mmol) and $NaCNBH_3$ (1.2 g; 19 mmol), pH was adjusted to 6.0 with acetic acid. Stirring was continued at room temperature for 3 days. The mixture was evaporated and the residue was made alkaline and extracted with $CH_2Cl_2$. After drying ($Na_2SO_4$) and evaporation the title compound as base was transformed to the HCl-salt. Yield: 1.1 g (83%). Mp: 289° dec.
$[\alpha]_D^{25}$ =-29° (C=0.015; MeOH)

**d) (R)-3-(N-Isopropyl-N-n-propylamino)-5-methoxychroman**

(R)-3-(N-Isopropylamino)-5-methoxychroman (17.5 g, 0.079 mol) was dissolved in dimethylformamide (DMF) (180 mL). $K_2CO_3$ (21.8 g, 0.18 mol) and 1-iodopropane (53.8 g, 0.32 mol) were added. The reaction mixture was then stirred at 50°C for 5 days. The solvent was removed in vacuo. The residue was dissolved in ether/$NH_3$ (1 M). The phases were separated and the water phase was washed once with ether. The combined ether-layers were dried ($MgSO_4$) and the solvent was removed in vacuo to give a yellow oily residue. The oil was dissolved in dry ether and title compound was obtained in form of the HC1-salt in 66% yield (15.7 g) by slow addition of HCl in ether at 0°C. Mp (HCl-salt): 108-120°C.
$[\alpha]_D^{25}$ =(Base)=-95.5°(C=0.1;MeOH).

**e) (R)-5-Hydroxy-3-(N-isopropyl-N-n-propylamino)chroman**

The HCl-salt of (R)-3-(N-isopropyl-N-n-propylamino)-5-methoxychroman (15.5 g, 52 mmol) was mixed with $CH_2Cl_2$ (100 mL) under nitrogen atmosphere and cooled to -60°C. $BBr_3$(27.2 g, 110 mmol) dissolved in $CH_2Cl_2$ (50 mL), was added slowly. The temperature was then raised to 0°C and the reaction mixture was stirred at 0°C for 12h. The reaction mixture was then slowly added to a stirred saturated $NaHCO_3$ solution. The layers were separated and the water layer was extracted once with $CH_2Cl_2$. The combined organic layers were dried ($MgSO_4$). Removal of the solvent in vacuo gave the phenolic title compound. Yield: 98%.
$[\alpha]_D^{22}$ (Base)=-83.0° (C=0.1;MeOH).Mp(HCl-salt): 215-222°C (decomposes).

**f) (R)-3-(N-Isopropyl-N-n-propylamino)-5-trifluoromethane sulfonyloxychroman (VI)**

(R)-5-Hydroxy-3-(N-isopropyl-N-n-propylamino)chroman (13 g, 52 mmol) was dissolved in $CH_2Cl_2$ (100 mL) under nitrogen atmosphere. 2,4,6-Collidine (8.2 g, 68 mmol) was added and the mixture was cooled to -60°C. Trifluoromethanesulfonic anhydride (17.7 g, 62 mmol) was added slowly over a one hour period. The temperature was then raised to 0°C and the reaction was quenched with saturated $Na_2CO_3$. The layers were separated. The water layer was pH adjusted with $NH_3$(2 M) to pH 8 and extracted once with $CH_2Cl_2$. The combined organic layers were dried ($MgSO_4$). Removal of solvent in vacuo gave a brown oily residue which was purified by flash chromatography $SiO_2$($CH_2Cl_2$) to give the triflate compound. Yield: 76% (15 g).
$[\alpha]_D^{22}$ =-77.9°(C=0.01;MeOH).

**g) (R)-3-(N-Isopropyl-N-n-propylamino)-5-(N-isopropyl)-carbamoylchroman (I)**

(R)-3-(N-Isopropyl-N-n-propylamino)5-trifluoromethanesulfonyloxychroman (5.2 g, 14 mmol) was dissolved in DMF (30 mL). Isopropylamine (10 mL) was added and the vessel was evacuated followed by inlet of CO-gas. This procedure was repeated twice before palladium (II) acetate (90 mg) and 1,3-bis(diphenylphosphino)propane (144 mg) was added. The reaction mixture was then stirred for 10h at 65°C. The solvent was removed in vacuo. The dark brown residue was dissolved in diethylether/$NH_3$ (1 M). The layers were separated and the water-phase was extracted once with ether. The combined ether layers were dried ($MgSO_4$). Removal of solvent in vacuo gave a yellow crystalline residue which was purified by flash chromatography $SiO_2$ ($CH_2CL_2$/EtOAc,10:1). Recrystallisation from $CH_2Cl_2$/hexane gave the pure amide (R)-3-(N-isopropylamino-N-n-propylamino)-5-(N-isopropyl)carbamoylchroman. Yield: 35% (1.5 g).
$[\alpha]_D^{21}$ =-87.0°(C=0.1;MeOH).Mp:94-95.4°C.

Example 3

**(R)-3-(N-Isopropylamino-N-n-propylamino)-5-(N-isopropyl)-carbamoylchroman (I) (Method B)**

**a) (R)-3-(N-Isopropylamino)-5-methoxychroman (V)**

5-Methoxy-3-chromanone (50 g, 0,28 mol; described in EP 0 222 996) was dissolved in methanol (300 mL). The solution was cooled to 0°C before the isopropylamine (70 mL, excess) was added. The pH was adjusted to about 6 by the addition of acetic acid. $NaCNBH_3$ (12 g, 0.19 mol) was added in portions during a one hour period and pH was kept at pH 6. The ice-bath was removed and the mixture was stirred overnight at room temperature. The solvent was removed in vacuo. The residue was dissolved in ether/$NH_3$ (1 M). The layers were separated and the amine in the ether layer was subjected to acid-base extraction (HCl(1M)/$NH_3$(1M)). Drying with $MgSO_4$ and evaporation of solvent gave an colorless crystalline product.
Yield: 87% (54 g). Mp: 255-56°C (HCl-salt of the racemic product).

The racemic 3-(N-isopropylamino)-5-methoxychroman (IV; 54 g, 0.224 mol) was mixed with an equimolar amount of (+)-di-1,4-toluoyl-D-tartaric acid (98.6 g, 0.244 mol). The mixture was dissolved in boiling ethanol (170 mL)/acetone (70 mL). The salt started to crystallize in the hot solvent mixture but was not filtered off until the solvent had cooled down to room temperature (20°C). The salt was then recrystallized nine times from ethanol/acetone (1:5) to give the pure diastereomeric salt. Yield: 35% (54 g) (99% e.e). Mp: 166-168°C. The free enantiomer as the base (R)-3-(N-isopropylamino)-5-methoxychroman was obtained by the extraction of the salt in ether/KOH (1M). Yield: 32%, 17.5 g,
$[\alpha]_D^{21}$=-32°(C=0.1;MeOH).
Mp. 285-286°C (HCl-salt)

**b) (R)-3-(N-Isopropyl-N-n-propylamino)-5-methoxychroman**

The title compound was prepared in the same way as described in Example 1b and obtained in the same amount and with the same physical data as given in Example 2d.

**c) (R)-5-Hydroxy-3-(N-isopropyl-N-n-propylamino)chroman**

The title compound was prepared in the same way as described in Example 2e and obtained in the same amount and with the same physical data as given in Example 2e.

**d) (R)-3-(N-Isopropyl-N-n-propylamino)-5-trifluoromethanesulfonyloxychroman (VI)**

The title compound was prepared in the same way as described in Example 2f and obtained in the same amount and with the same physical data as given in Example 2f.

**e) (R)-3-(3-N-Isopropyl-N-n-propylamino)-5-(N-isopropyl)carbamoylchroman (I)**

The title compound was prepared in the same way as described in Example 2g and obtained in the same amount and with the same physical data as given in Example 2g.

Example 4

**a) (R)-3-(N-Isopropyl,N-n-propylamino)-5-methoxycarbonylchroman**

(R)-3-(N-Isopropyl,N-n-propylamino)-5-trifluoromethanesulfonyloxychroman (4.0 g, 10.5 mmol), triethylamine (2.3 g, 23.1 mmol) and a mixture of DMF/MeOH (18 mL, 6:2) was mixed in a three necked round-bottom flask. The flask was evacuated followed by inlet of CO-gas (repeated two times). Finally 1,3-bis(diphenylphosphino)propane (0.11 g) and palladium(II)acetate (0.07 g) was added. The mixture was stirred at 70°C for 17 hours. The reaction mixture was diluted with diethyl ether, washed with 2M $NH_3$ and dried ($MgSO_4$). Removal of solvent in vacuo gave a brown oily residue which was purified by flash chromatography ($SiO_2$, $CH_2Cl_2$/EtOAc; 10:1) to give the title compound in 82% yield (2.5 g).
$[\alpha]_D^{21}$=-131.6° (MeOH, 0.1M).
GC-MS(70eV)=291($M^+$), 262 (100%)

**b) (R)-3-(N-Isopropyl,N-n-propylamino)-5-N-isopropyl)-carbamoylchroman (I)**

(R)-3-N-(N-Isopropyl,N-n-propylamino)-5-methoxycarbonylchroman (0.46 g, 1.6 mmol), dissolved in MeOH (10mL), was mixed with a solution of NaOH (0.06 g, 1.6 mmol) in water (3 mL). The reaction mixture was then refluxed for 3.5 hours before the solvent was removed in vacuo. The residue was dissolved in toluene. The toluene was removed in vacuo (repeated two times) in order to form an azeotrope to remove the water. The residue was then dissolved in $SOCl_2$ (5 mL) and refluxed for 1 hour. The excess of $SOCl_2$ was removed in vacuo. The residue was dissolved in $CH_2Cl_2$ (20mL, dried with molecular sieves 3Å) before 4 mL isopropylamine was added. The reaction mixture was stirred for 1 hour at room temperature (21°C) before it was diluted with diethyl ether, washed (2M $NH_3$) and dried ($MgSO_4$). Removal of solvent in vacuo gave a yellow oily residue which was purified by flash chromatography ($SiO_2$, $CH_2Cl_2$/EtOAc; 5:2) to give the pure title compound in 88% yield (0.46 g).
$[\alpha]_D^{21}$ =-90.4°(MeOH 0.1).Mp:93-94°C.

Pharmacology

Pharmacological treatment of depression in man

Evidence is available that in depressed patients the neurotransmission in the central nervous system (CNS) may be disturbed. These disturbances appear to involve the neurotransmitters noradrenaline (NA) and 5-hydroxytryptamine (5-HT). The drugs most frequently used in the treatment of depression are considered to act by improving the neurotransmission of either or both of these physiological agonists. Available data suggest that the enhancement of 5-HT neurotransmission will primarily improve the depressed mood and anxiety, whereas the enhancement of noradrenaline neurotransmission will rather improve the retardation symptoms occurring in depressed patients. In recent years many efforts have been made to develop new drugs with high selectivity for improvement of 5-HT neurotransmission in the CNS.

The dominating mechanism of action for the drugs generally used today in the therapy of mental depression is by blocking the reuptake of the endogenous neurotransmitters (NA and/or 5-HT) released from nerve terminals in the CNS, thus increasing the concentration of these transmitters in the synaptic cleft and hence restoring an adequate neurotransmission.

A fundamentally different way to improve the neurotransmission in the central 5-HT-neurons would be to use a direct 5-HT-receptor agonist. In order to minimize side effects, a high selectivity for this kind of receptors would then be preferable.

Surprisingly, we have found that the racemate as well as the (R)-enantiomer of the compound 3-(N-isopropyl-N-n-propylamino)-5-(N-isopropyl)carbamoylchroman has show high stereo selective, direct stimulating effect on the $5\text{-}HT_{IA}$-receptors in CNS combined with a good bioavailability.

In vitro test: Receptor binding assay

In order to evaluate the 5-HT-receptor stimulating effect and selectivity, the affinity for various receptors in rat brain were measured in vitro using receptor assay. The stereospecific (R)-enantiomeric compound of the invention (R)-3-(N-isopropyl-N-n-propylamino)-5-(N-isopropyl)carbamoylchroman has $K_i$ 8.6 (nM) and the racemic compound 3-(N-isopropyl-N-n-propylamino)-5-(N-isopropyl)-carbamoylchroman has $K_i$ 24 (nM). The $K_i$ valve for the (S)-enantiomer exceeded 1000 nM.

Method: $5HT_{1A}$ binding assay. Cerebral cortex + hippo-campus from each rat was dissected and homogenized in 15 ml ice-cold 50 mM Tris-HCl buffer 4.0 mM $CaCl_2$ and 5.7 mM ascorbic acid, pH 7.5 with an Ultra-Turrax (Janke & Kunkel, Staufen, FRG) for ten s. After centrifugation for 12.5 min at 17,000 rpm (39,800 x g in a Beckman centrifuge with a chilled JA-17 rotor (Beckman, Palo Alto, CA, USA), the pellets were resuspended in the same buffer and homogenization and centrifugation repeated. To each pellet 5 ml ice-cold 0.32 M sucrose were added and homogenized for 5 sec. These samples were kept frozen at -70°C. When used they were diluted with the buffer to 8 mg tissue/ml and homogenized for 10 sec. The tissue homogenates were incubated for ten minutes at 37°C and then supplied with 10 µM pargyline followed by reincubation for 10 minutes.

The binding assay followed that described by Peroutka, J. Neurochem. 47, 529-540, (1986). The incubation mixture (2 ml) contained $^3H$-8-OH-DPAT (0.25 to 8 nM), 5 mg/ml tissue homogenate in 50 mM Tris-HCl buffer containing 4.0 mM $CaCl_2$ and 5.7 mM ascorbic acid, pH 7.5. Six different concentrations of $^3H$-8-OH-DPAT were analyzed. Binding experiments were started by the addition of tissue homogenate and followed by incubation at 37°C for 10 minutes. The incubation mixtures were filtered through Whatman GF/B glass filters with Brandel Cell Harvester (Gaithersburg, MD, USA). The filters were washed twice with 5 ml ice-cold 50mM Tris-HCl buffer, pH 7.5, and counted with 5 ml Ready-solv HP (Beckman) in a Beckman LS 3801 scintillation counter. Non-specific binding was measured by the addition of 10 µM 5-HT to the reaction mixture. The binding data was processed by non-linear least squares computer analysis (Munson and Rodbard, Anal. Biochem. 107, 220-239, (1980)).

In vivo test: Oral bioavailability in dogs

The bioavailability test was performed as described below and gives the mean value of 17% for the (R)-enantiomer and 21% for the racemate of compound 3-(N-isopropyl-N-n-propylamino)-5-(N-isopropyl)carbamoylchroman based on plasma level measurements in dogs. Dose-effect studies in rat following subcutaneous versus oral administration further support a high availability of the compound at the receptor after oral administration.

Method: Assessment for oral bioavailability (systemic availability) was based on the plasma area under the curve (AUC) method. An aqueous saline solution of compound of the invention was administered intravenously (i.v.) and orally (p.o.) to the animals and concentrations of the compound in plasma measured at numerous timepoints. The doses administered were 1 $\mu mol \cdot kg^{-1}$ and 10 $\mu mol \cdot kg^{-1}$ for intravenous and oral administration, respectively. AUC was calculated according to the trapezoidal rule. Determination of the test compound in plasma was accomplished by an HPLC method which incorporated electrochemical detection.

In vivo test: Synthesis of 5-HT

As predicted for a selective $5\text{-}HT_{IA}$ agonist, the synthesis rate of 5-HT, measured as a significant decrease of the 5-HTP level in rat brain was recorded after 0,1 mg/kg following subcutaneous administrations as well as after 0,2 mg/kg oral administration of the (R)-enantiomer of compound 3-(N-isopropyl-N-n-propylamino)-5-(N-isopropyl)carbamoylchroman .

Method: The rate of synthesis of 5-HT in the rat streatum was measured as the accumulation of 5-HTP during 30 min after inhibition of aromatic L-amino acid decarboxylase by NSD 1015 (decarboxylase inhibitor, 100 mg/kg i.p.). The test compound was administered 30 min before the NSD 1015. The regions of the brain to be examined were dissected, frozen and stored.

The levels of 5-HTP (5-hydroxytryptophan) was determined by use of high performance liquid chromatography (HPLC) with electrochemical detection according to the method of Magnusson, Nilsson and Westerlund (1980). The mobile phase was 0,1 M phosphate buffer (pH 2,5):methanol:acetonitrile-89:9:2 v/v, containing 1mM octylsulphate. The frozen samples were weighed and homogenized in 0,1 M perchloric acid, containing 2,5 nM sodium bisulphite, 1 mM ethylene diamine tetraacetic acid (EDTA) and epinine as an internal standard. The supernatants were injected directly onto a Supelcosil $C_{18}$ (3 μM) column, connected to a detector (ESA Coulochem 5100A), set to 0,05/0,40 V.

Temperature effects

A significant temperature decrease was obtained in rats following subcutaneous administration of 0.3 mg/kg or 1 mg/kg using oral administration of the (R)-enantiomer of compound 3-(N-isopropyl-N-n-propylamino)-5-(N-isopropyl)carbamoylchroman.

Method: In each test, thirty rats, weighing approx 250 g, housed in 6 cages of 5 rats, are used. The rats have free access to food and water. Before the start of testing, they are numbered and left undisturbed for at last 1 hour. Before the administration of the compound, the body temperature of each rat is measured using a YSI 2100 tele-thermometer. The thermometer probe is inserted 10 cm into the rectum and left in pace for thirty seconds. The drug is then administered either subcutaneously or orally. In each experiment vehicle and 4 doses of drug are tested. One rat in each cage receives each treatment. The order of treatment is rotated since disturbance to the cage increases the activity of the rats, and thereby their body temperature. Thirty minutes after drug administration the rats' body temperature are measured again. The procedure is repeated 60, 90 and 120 minutes after drug administration. The resultant data on body temperature is subjected to analysis of variance. A significant group by time interaction is taken as an indication of drug effect. To obtain the minimum effective dose, the mean temperature for each of the drug treated groups are compared with that of the vehicle group at each time point using Dunnett's t-test with a level of significance of $p<0.02$. An indication of bioavailability may be obtained by calculating the ratio between the minimum effective doses following oral and subcutaneous administration.

## Claims

1. A process for preparing (R)-3-amino-5-methoxychroman by dissolving racemic 3-amino-5-methoxychroman and L(+)tartaric acid in water, heating the solution until a clear solution is obtained and let said clear solution crystallize by obtaining room temperature.

2. (R)-3-Amino-5-methoxychroman prepared by the process described in claim.